# EUROPÄISCHE PATENTSCHRIFT

(11) **EP 4 103 098 B1**
(45) Veröffentlichungstag und Bekanntmachung des Hinweises auf die Patenterteilung: **02.12.2026**
(21) Anmeldenummer: 21836581.5
(22) Anmeldetag: 22.12.2021
(51) Int. Cl.: A61B 46/10, A61B 90/50, A61B 90/57

(54) **POSITIONIERARM**
POSITIONING ARM
BRAS DE POSITIONNEMENT

(30) Priorität: 29.12.2020 DE 202020107591 U
(43) Veröffentlichungstag der Anmeldung: 21.12.2022
(73) Patentinhaber: Isys Medizintechnik GmbH, 6370 Kitzbühel (AT)
(72) Erfinder: VOGELE, Michael, 86830 Schwabmünchen (DE)
(74) Vertreter: Keller Schneider Patentanwaltsgesellschaft mbH
(86) Internationale Anmeldenummer: PCT/EP2021/087291
(87) Internationale Veröffentlichungsnummer: WO 2022/144273

(56) Entgegenhaltungen:
- EP-A2- 2 085 186
- ES-T3- 2 277 375
- JP-U- S59 161 028
- US-A- 4 461 284
- US-A1- 2020 085 530
- US-A1- 2020 163 658
- US-B2- 7 294 140
- HELMUT KAPCZYNSKI: "Surgical Instruments 101 An Introduction to KMedic Certified Instruments", INTERNET CITATION, 1 January 1997 (1997-01-01), pages 1 - 181, XP008172530, Retrieved from the Internet <URL:http://www.teleflex.com/en/usa/pdf/KMedic_Surgical_Instruments_101.pdf> [retrieved on 20141003]

## Beschreibung

### Technisches Gebiet

Die vorliegende Erfindung betrifft einen Positionierarm zum Positionieren von Instrumenten, insbesondere medizintechnischen Instrumenten. Mittels des erfindungsgemäßen Positionierarms lassen sich jedoch auch eine Vielzahl von anderen Instrumenten, Geräten oder Werkzeugen exakt positionieren. Dies können beispielsweise Messinstrumente, Monitore, Mikrofone, Lautsprecher und Beleuchtungskörper sein. Auch zur Halterung von Gefäßen und Geräten in Laboren sind derartige Positionierarme bestens geeignet.

### Stand der Technik

Aus dem Stand der Technik sind für unterschiedliche Anwendungen verschiedenste Positionierarme mit unterschiedlichsten Anforderungen bekannt. Insbesondere bei Positionierarmen zum Positionieren von medizintechnischen Instrumenten werden beispielsweise sehr hohe Anforderung an die Stabilität, Zuverlässigkeit und Bedienbarkeit der Positionierarme gestellt. So sind im Stand der Technik zahlreiche Versuche unternommen worden, Positioniersysteme unter Berücksichtigung dieser Anforderungen weiterzuentwickeln und zu verbessern.

Beispielsweise ist aus der Druckschrift WO 2017 / 144 172 A1 der Anmelderin ein Positionierarm mit einem Drehkörper bzw. Drehgelenk und zwei daran angelenkten Armelementen bekannt. Dabei ist der Winkel zwischen den beiden Armelementen durch eine an dem Drehkörper über eine Kupplung angebrachte Ratsche feststellbar, wobei an der Kupplung ein Schalter vorgesehen ist, der die Winkelposition der Arme in einer gewünschten Schalterstellung zueinander verriegelt.

Der aus der Druckschrift WO 2017 / 144 172 A1 bekannte Positionierarm hat den Vorteil, dass mittels der Ratsche einfach und mit verhältnismäßig geringem Kraftaufwand ein Feststellen d.h. Blockieren des Drehgelenks des Positionierarms möglich ist. Insbesondere ist die Ratsche auch in einem sterilen Umfeld, beispielsweise unter einer sterilen Abdeckfolie, gut bedienbar. Außerdem kann mittels der Ratsche ein bestimmtes Mindest-Drehmoment vorgegeben werden, so dass das Blockieren des Positionierarms zuverlässig erfolgen kann.

Allerdings hat der aus der WO 2017 / 144 172 A1 bekannte Positionierarm immer noch den Nachteil, dass die Bedienung nach wie vor vom Geschick eines Benutzers abhängig ist. So bleibt ein Restrisiko bei der Anwendung, wenn der Anwender die Ratsche nicht richtig betätigt und sich der Arm während eines operativen Eingriffs verstellt. Außerdem ist die Ergonomie bzw. Gebrauchstauglichkeit nach wie vor nicht optimal. So muss der Anwender beispielsweise beim Positionieren des Positionierarms gleichzeitig das Instrument am Ende des Positionierarms halten und das Operationsfeld im Auge behalten. Solch ein simultaner Ablauf ist für den Anwender sehr schwierig, insbesondere, da beim Feststellen des Arms relativ hohe Kräfte aufgewendet werden müssen und die Rotationsbewegung beim Drehen der Ratsche mit Bewegungen der anderen Hand konträr laufen kann. Da der Positionierarm vor, während und nach eines chirurgischen Eingriffs vielfach umpositioniert werden muss, d.h. vielfaches Öffnen und Schließen erforderlich ist, wäre zudem eine schnellere Bedienmöglichkeit wünschenswert.

Ein weiterer Positionierarm ist aus dem US-Patent US 10 687 915 B2 bekannt. Bei diesem wird ein Druck zum Arretieren von Gelenken über hydraulisch, pneumatisch oder mechanisch verstellbare Kolben und/oder Gestänge auf die Gelenke übertragen. Dies erfordert hohe Schließkräfte und relativ komplexe Übertragungsorgane.

Die bekannten Drei-Gelenk-Haltearme bzw. Drei-Gelenk-Stative bestehen aus einer Vielzahl von Bauelementen, die oftmals sehr filigran sind und sehr hohe Druck- und Biegekräfte aushalten müssen. Dadurch ist die Produktion teuer und man ist bei der Materialauswahl sehr eingeschränkt. Es müssen harte Metalllegierungen oder spezielle gehärte Stähle insbesondere bei den Druckelementen/Druckstangen und in den Gelenken verwendet werden (teilweise mit Spezialbeschichtungen) weil andere Materialen nicht standhalten. Die Verwendung von leichten Alu- oder Kunststoffmaterialen (für bessere Handhabung oder für die Verwendung im Röntgenstrahl oder Magnetfeld eines Kernspintomographen) geht daher bei den bekannten Drei-Gelenk-Haltearmen bzw. Drei-Gelenk-Stativen immer mit Verlust des Verriegelungsdrucks in den Gelenken und Verlust der Haltekraft des Arms einher, ebenso wird die Haltbarkeit reduziert.

Die Dokumente US 4 461 284 A, US 2020/163658 A1, US 2020/085530 A1, US 7 294 140 B2, HELMUT KAPCZYNSKI: "Surgical Instruments 101 An Introduction to KMedic Certified Instruments" (1. Januar 1997 (1997-01-01), Seiten 1-181, XP008172530), ES 2 277 375 T3, EP 2 085 186 A2 und JP S59 161028 U offenbaren weiteren relevanten Stand der Technik. Ausgehend von dem vorstehend aufgeführten Stand der Technik ist es Aufgabe der vorliegenden Erfindung, einen Positionierarm zur Verfügung zu stellen, der die Probleme und Nachteile der aus dem Stand der Technik bekannten Systeme behebt und entsprechende Vorteile gegenüber diesen hat. Insbesondere ist es Aufgabe der vorliegenden Erfindung, einen Positionierarm anzugeben, der eine ergonomisch vorteilhafte, komfortable und schnelle Bedienung des Positionierarms erlaubt.

### Darstellung der Erfindung

Diese Aufgabe wird durch den Gegenstand des unabhängigen Anspruchs 1 gelöst. Weitere mögliche Ausgestaltungen der Erfindung sind insbesondere in den abhängigen Ansprüchen angegeben.

Die erfindungsgemäße Lösung sieht einen Positionierarm zum Positionieren von Instrumenten oder Werkzeugen vor, der mindestens zwei Armelemente aufweist, die mittels eines zentralen Gelenks um eine Schwenkachse schwenkbar miteinander verbunden sind. Dabei weist zumindest eines der Armelemente an einem dem zentralen Gelenk entgegengesetzten Ende ein weiteres Gelenk auf. Der Positionierarm weist weiterhin eine Blockiereinrichtung zum Blockieren und Freigeben des zentralen Gelenks und des zumindest einen weiteren Gelenks auf, die am zentralen Gelenk angeordnet ist und über zumindest eine an den Armelementen oder von diesen gebildete Übertragungseinrichtung das Blockieren und Freigeben der weiteren Gelenke bewirkt. Durch die Anordnung der Blockiereinrichtung am zentralen Gelenk erfolgt die Betätigung in einem ergonomisch günstigen Griffbereich seitlich oberhalb des Patienten.

Der erfindungsgemäß besonders einfach aufgebaute, robuste Positionierarm zeichnet sich dadurch aus, dass die Übertragungseinrichtung von wenigstens einem Paar von Zangenarmen und besonders bevorzugt von zwei Paaren von Zangenarmen gebildet wird. Die Zangenarme weisen jeweils einen längeren Hebelarm und einen kürzeren Greifarm auf, wobei die Hebelarme mit ihren hinteren Enden fluchtend im Bereich des zentralen Gelenks mittels der Blockiereinrichtung verbindbar sind und die Greifarme einen Teil der weiteren Gelenke bilden. Dieser Ausführungsform benötigt sehr geringe Betätigungskräfte, weil durch die Zangenarme in Ergänzung zur Schließkraft der Blockiereinrichtung eine zusätzliche Hebelübersetzung zwischen der Krafteinleitungsstelle an den Enden der längeren Hebelarme und der Kraftausleitungstelle an den kürzeren Greifarmes erfolgt.

Durch die erfindungsgemäße Ausführungsform wird ein Gelenkarm mit drei Hauptgelenken geschaffen, der anders als die bekannten Gelenkarme nicht mit filigranen, zusätzliche Führungen erfordernden Druckelementen im Innern der Armelemente arbeitet, sondern mit robusten, die Armelemente unmittelbar bildenden Zangenpaaren.

Bevorzugt weisen beide Armelemente jeweils ein weiteres Gelenk auf und die Blockiereinrichtung ist mittels jeweils einer Übertragungseinrichtung mit dem weiteren Gelenk verbunden.

Der Positionierarm dient insbesondere dazu, während eines Eingriffs an einem Patienten medizinische Instrumente, Werkzeuge oder Hilfsmittel zuverlässig, d.h. sicher und präzise zu positionieren bzw. zu halten. Insbesondere bei neurochirurgischen oder bioptischen Eingriffen ist ein solch zuverlässiges Positionieren der Instrumente bzw. Werkzeuge, beispielsweise Nadeln, Haken, Pinzetten oder Markern unerlässlich.

Bei der Verwendung des Positionierarms zum Positionieren medizinischer Instrumente kann beispielsweise eines der Armelemente über ein weiteres Gelenk mit einer Befestigungseinrichtung für einen OP-Tisch verbunden ausgebildet sein. Das andere Armelement kann entsprechend über ein weiteres Gelenk mit einem besonders vorteilhaft als Universal-Adapter gestalteten Instrumentenhalter verbindbar ausgebildet sein. Anstelle einer Befestigungsvorrichtung für einen OP-Tisch kann ein äußeres Ende eines der Armelemente auch mit einer Magnetspannvorrichtung zur Befestigung an einer metallischen Fläche oder mit einer anschraubbaren Flanschplatte zur vorübergehenden oder dauerhaften Befestigung an einer Wand oder einem Träger versehen sein.

Gemäß einer ersten bevorzugten Ausführungsform weist das wenigstens eine weitere Gelenk einen drehbaren Klemmkörper auf. Der drehbare Klemmkörper ist entweder als Kugel ausgebildet oder als Drehhülse, wobei letztere besonders vorteilhaft zur zusätzlichen Lagerung einer drehbaren Kugel ausgebildet ist und dadurch neben einer beliebigen Drehung auch einen nahezu beliebigen Schwenkwinkel erlaubt.

Der einfache robuste Aufbau aus wenigen Teilen, von denen jeweils zumindest zwei Zangenarme als Gleichteile kostengünstig herstellbar sind und die einfache Montierbarkeit ermöglichen auch eine sichere und effiziente Sterilisation aller Teile des Positionierarms, während bekannte Positionierarme eine Vielzahl von Teilen mit zahlreichen Hinterschnitten, Bohrungen, Sacklöchern, Verklebungen oder dergleichen aufweisen und dadurch für einen Sterilisationsprozess nur schwer zerlegbar und schwer in allen Bereichen zugänglich sind.

Erfindungsgemäße Positionierarme zeichnen sich neben der einfachen Herstellbarkeit und leichten Montierbarkeit auch dadurch aus, dass sie bei geringem Gewicht auch hohe Traglasten sehr sicher und millimetergenau fixieren können. Da die verwendeten Zangenarme auch sehr einfach aus Kunststoff herstellbar sind, sind die von ihnen gebildeten Positionierarme beispielsweise auch bei MRT-Anwendungen im Strahlenbereich der Röhre unmittelbar verwendbar.

Die Blockiereinrichtung weist bevorzugt ein in axialer Richtung des zentralen Gelenks verlagerbares, mit einer Betätigungsvorrichtung verbundenes Betätigungselement auf.

Die Betätigungsvorrichtung kann in einer einfacheren Ausführungsform von einem mit einem Gewinde verbunden Drehgriff, von einem mit einem Exzenter verbundenen Spanngriff oder von einem vorzugsweise batteriebetriebenen elektrischen Motor gebildet sein. Obwohl das Blockieren mittels der Blockiereinrichtung bevorzugt elektrisch erfolgt, kann alternativ dazu auch ein manuelles Blockieren bzw. Freigeben der Gelenke möglich sein. So kann die Blockiereinrichtung beispielsweise über ein Gewinde oder einen Exzenter auch ohne elektrische Energie betrieben werden. In Verbindung mit einer Ausbildung der Übertragungseinrichtung durch Zangenarme erfolgt eine zusätzliche mechanische Übersetzung zwischen Kraft Einleitung und Kraftausleitung, die etwa im Verhältnis 1:2 bis 1:10 liegen kann. Durch diese zusätzliche Übersetzung sind an der Blockiereinrichtung nur relativ geringe Betätigung nötig Die im Falle eines motorischen Antriebs zum Betrieb der Blockiereinrichtung benötigte elektrische Energie wird vorzugsweise mittels eines Akkumulators geliefert. So ist keine Verkabelung nötig, die innerhalb des OP-Raums eine Gefahrenquelle darstellen kann.

Mit dem erfindungsgemäßen Positionierarm wird die Aufgabe in hervorragender Weise gelöst. Sowohl mit einer manuellen Betätigungsvorrichtung als auch mit einer motorisierten Betätigungsvorrichtung ist die Blockiereinrichtung sehr komfortabel, einfach und schnell bedienbar. Außerdem ist die Bedienung zumindest im Wesentlichen unabhängig von den verfügbaren Kräften eines Benutzers ausgebildet. Durch die zusätzliche Übersetzung mittels der Übertragungseinrichtung kann der Positionierarm auch mit geringem Kraftaufwand stets stabil genug arretiert werden und sich während des Betriebs nicht verstellen. Ferner ermöglichen die geringen bedienen Wege der Blockiereinrichtung eine sehr schnelle Bedienung. Ein vollständiges Blockieren der Gelenke des Positionierarms kann innerhalb weniger Sekunden erreicht werden.

Ein weiterer Vorteil der rein mechanisch oder elektrisch betriebenen Blockiereinrichtung liegt in ihrer Kompaktheit. Bei elektrischen Antrieben gab es in den vergangenen Jahren enorme technische Weiterentwicklungen. Alternative bekannte Drucksysteme, die mit Druckluft, Hydrauliköl oder dergleichen arbeiten (siehe beispielsweise die eingangs erwähnte US 10 687 915 B2) sind kostenintensiv in der Herstellung, komplex in der Anwendung und Wartung, und stellen aufgrund der verwendeten Flüssigkeiten für das sterile Operationsfeld und den Anwender ein durch die Erfindung vermeidbares Risiko dar. Gerade hinsichtlich der Sterilität ist vorteilhaft, dass elektrische Antriebe d.h. Motoren inklusive der dazugehörigen Elektronik mittlerweile auch sterilisierbar, insbesondere autoklavierbar, verfügbar sind. Dies ist insbesondere bei Anwendungen relevant, bei denen man nicht mit einer sterilen Abdeckfolie arbeiten kann. Bezüglich der Sterilisierbarkeit zeichnet sich der gesamte erfindungsgemäße Positionierarm dadurch aus, dass dessen wenige Teile zum Reinigen sehr einfach und schnell vollständig demontierbar und nach dem Sterilisieren auch einfach wieder zusammenfügbar sind.

Insbesondere das progressive Öffnen der einzelnen Gelenke kann erwünscht sein, um zu verhindern, dass es nur einen vollständig geöffneten Zustand gibt, in dem sich alle Gelenke des Positionierarms instabil hin und her bewegen. Durch eine entsprechende Abstimmung und Einstellung der Betätigungswege der Blockiereinrichtung, der Übertragungseinrichtungen und der in den weiteren Gelenken verwendeten Klemmkörper kann in einfacher Weise exakt festgelegt werden, welches der Gelenke zu Beginn der Betätigung der Blockiereinrichtung als erstes und welches als letztes vollständig verriegelt. Durch Verwendung von Klemmkörpern mit geringfügig unterschiedlichen Durchmessern oder durch eine Verwendung von Distanzscheiben im Bereich des zentralen Gelenks lässt sich diese Reihenfolge bei Bedarf auch nachträglich verändern.

Das weitere Gelenk weist bevorzugt einen drehbaren Klemmkörper auf. Eine erste vorteilhafte Ausführungsform sieht vor, dass das weitere Gelenk mit einer Kugel als Klemmkörper als Kugelgelenk ausgebildet ist. Ein Kugelgelenk ist ein Gelenk mit einer frei dreh- und schwenkbaren Kugel. Die durch Zangenarme gebildeten Übertragungseinrichtungen können hier beispielsweise eine Kugel unmittelbar zwischen ihren bevorzugt an der Innenseite ihrer Enden mit leicht konkaven Vertiefungen muldenartig geformten Greifarmen aufnehmen und beim Zusammendrücken der Hebelarme arretieren.

Gemäß einer weiteren vorteilhaften Ausführungsform weist das weitere Gelenk eine Drehhülse auf, die an den Greifarmen der Zangenarme beispielsweise mittels einer Nut oder Eindrehung drehbar gehalten und beim Schließen der Greifarme arretiert wird.

Besonders vorteilhaft ist in einer Drehhülse an einem von den greifarmenabgewandten Ende eine aus mehreren Segmenten bestehende geschlitzte Aufnahme für eine Kugel eines Kugelgelenks vorgesehen, sodass bei nicht arretieren Greifarmen ein Drehen der Drehhülse und zusätzlich eine Schwenkbewegung der Kugel in der Drehhülse für zusätzliche Freiheitsgrade sorgt. Beim Arretieren der Greifarme wird gleichzeitig die Verdrehung der Drehhülse unterbunden und auch die Kugel durch Zusammenpressen der Segmente der Drehhülse fest arretiert.

Eine weitere vorteilhafte Ausführungsform sieht vor, dass der Klemmkörper mittels einer Zugstange mit einem zwei Zangenarme verbindenden Verbindungsgelenk (113; 123) verbunden ist. Die Zugstange zieht in diesem Fall den Klemmkörper bei einem Arretieren der Zangenarme gegen einen muldenförmigen Sitz an deren Ende.

Gemäß einer vorteilhaften Weiterbildung der Erfindung weist eine elektrische Blockiereinrichtung einen Elektromotor mit einer Ausgangswelle auf, die senkrecht zu der Achse des zentralen Gelenks ausgerichtet und mittels eines Kegelradantriebs oder Schneckengetriebes mit der Blockiereinrichtung verbunden ist. Bei einer derartigen Ausbildung liegt der Vorteil in einer besonders hohen Kompaktheit. Die elektrische Blockiereinrichtung kann platzsparend an einem der Armelemente angeordnet sein. Üblicherweise liegen bei den meisten Anwendungen nur sehr begrenzte Platzverhältnisse vor und ein bestmöglichster Zugang vom Operateur zu dem Operationsfeld ist von hoher Relevanz. Eine derartige, auch für die vorliegende Erfindung verwendbare Motor-Getriebe-Anordnung ist bereits in der älteren Anmeldung DE 10 2020 122 352.8 der Anmelderin vom 26.08.2020 beschrieben, die am 22.06.2021 auch als PCT/EP2021/067028 angemeldet wurde .

Sämtliche der vorab beschriebenen Vorteile können bei einem Positionierarm zum Positionieren von medizintechnischen Instrumenten besonders gut genutzt werden. Anstelle einfacher medizinischer Geräte und Instrumente können mittels des Positionierarms auch miniaturisierte Roboter in einer dem Anwendungsbereich nahen Stellung positioniert werden, wobei dann die weitere Betätigung wenigstens eines Instruments - wie beispielsweise einer Punktionsnadel - mittels eines mit mehreren Freiheitsgraden bewegbaren Roboters bevorzugt ferngesteuert erfolgt.

### Kurze Beschreibung der Figuren

Weitere Merkmale, Vorteile und Ausführungsformen der Erfindung ergeben sich aus der nachfolgenden Beschreibung anhand der Figuren. Es zeigen:
- Fig. 1: eine perspektivische Darstellung eines erfindungsgemäßen Positionierarms mit zwei in einem zentralen Gelenk verbundenen, jeweils aus einem Paar von Zangenarmen gebildeten Armelementen, die jeweils ein weiteres außen liegendes Gelenk aufweisen, wobei die Klemmkörper der außen liegenden Gelenke von Drehhülsen und in diesen aufgenommene Kugeln gebildet sind,
- Fig. 2: eine Variante zur Fig. 1, bei der die Klemmkörper der außen liegenden Gelenke von Kugeln gebildet werden und an einem der Außengelenke an einem Universal-Adapter ein als Haken ausgebildetes Instrument angeordnet ist,
- Fig. 3: die in Figur 2 gezeigte Variante mit einer zentralen Verrastung im Bereich des zentralen Gelenks,
- Fig. 4: die in Figur 2 gezeigte Variante mit zwei dezentralen Verrastungen im Bereich des zentralen Gelenks,
- Fig. 5: eine Variante zu den Figuren 1 bis 4, bei der die Blockiereinrichtung des zentralen Gelenks anstelle eines Drehgriff von einem mit einem Exzenter verbundenen Spanngriff betätigbar ist,
- Fig. 6: die in Figur 5 gezeigte Variante mit geöffnetem Spanngriff,
- Fig. 7: einen Längsschnitt durch das zentrale Gelenk mit einem Drehgriff und zwei als Tellerfedern ausgebildeten Federelementen,
- Fig. 8: eine Variante zu Figur 7 mit einem Spanngriff und einem zentral angeordneten, als Spiralfeder ausgebildeten Federelement,
- Fig. 9: die vier die beiden Armelemente bildenden Zangenarme nebeneinander in einer perspektivischen Ansicht von der Seite des zentralen Gelenks her,
- Fig. 10: die in Figur 9 dargestellten Zangenarme nebeneinander in einer perspektivischen Ansicht von der Seite der weiteren Gelenke her,
- Fig. 11: eine Variante zu den in Figur 9 und 10 gezeigten Zangenarmen, mit separat ausgebildeten, mit den Zangenarmen formschlüssig verbindbaren Rastelementen,

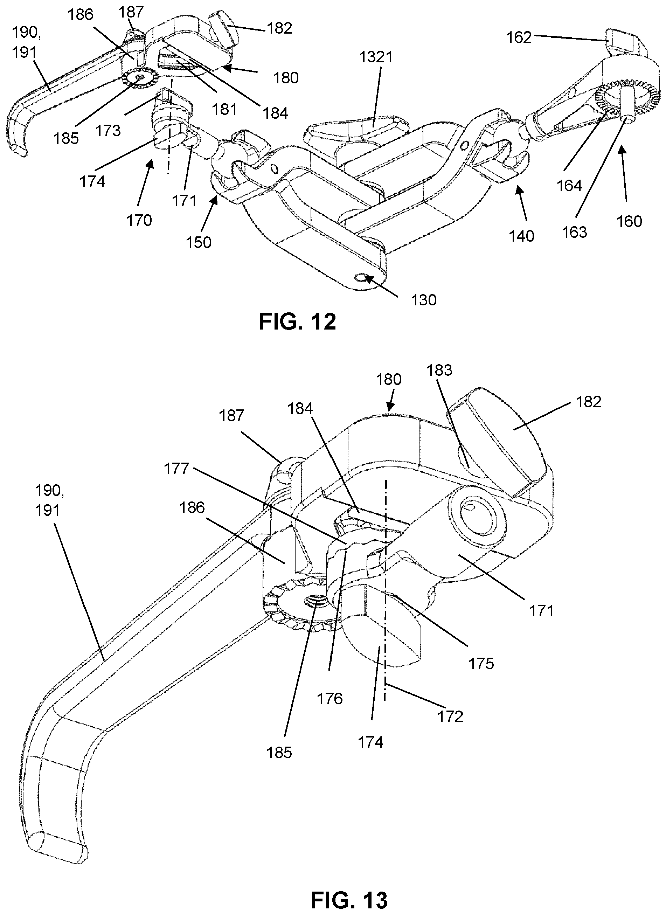

- Fig. 12: die in Figur 2 gezeigte Variante in einer perspektivischen Ansicht von unten mit vom Universal-Adapter abgenommenen Halter für das hakenförmige Werkzeug,
- Fig. 13: die Verbindung des Universal-Adapter und des Halters aus Figur 12 in vergrößerter Darstellung,
- Fig. 14: eine Drehhülse gemäß Figur 1 in vergrößerter Darstellung,
- Fig. 15-17: mit einem in Figur 15 im Eingriff befindlichen Universal-Adapter koppelbare unterschiedliche Halter für unterschiedliche Instrumente bzw. Werkzeuge,
- Fig. 18: eine Variante zu Figur 1 mit einem im Bereich des zentralen Gelenks angeordneten, die Armelemente federnd vorbelastenden Federelement, das beispielhaft als Drehschenkelfeder gestaltet ist, wobei zur Verdeutlichung einer der Zangenarme im Bereich des zentralen Gelenks entfernt wurde,
- Fig. 19: die Variante gemäß Figur 18 mit vollständig dargestellten Zangenarmen und einer im Bereich des Universal-Adapter angedeuteten sterilen Hülle bzw. Folie,
- Fig. 20: eine Variante zur Figur 4 mit einer motorischen Betätigung der Blockierervorrichtung im Bereich des zentralen Gelenks,
- Fig. 21: eine Variante mit einer in einem Klemmkörper gelagerten und an einem Verbindungsgelenk der Zangenarme befestigten Zugstange, wobei ein Zangenarm weggelassen wurde,
- Fig. 22: die Variante gemäß Fig. 21 mit beiden Zangenarmen,
- Fig. 23: die Variante gemäß Fig. 21 in Explosionsdarstellung in einer ersten Perspektive,
- Fig. 24: die Variante gemäß Fig. 22 in Explosionsdarstellung in einer zweiten Perspektive,
- Fig. 25: einen Positionierarm mit einer am äußeren Ende des ersten Armelements angeordneten Magnetbefestigung,
- Fig. 26: einen Positionierarm mit einer am äußeren Ende des ersten Armelements angeordneten Flanschplatte, und
- Fig. 27: eine Variante mit verkürzten Zangenarmen.

### Wege zur Ausführung der Erfindung

Ähnliche Elemente sind in den Figuren allgemein mit gleichen oder ähnlichen Bezugsziffern versehen.

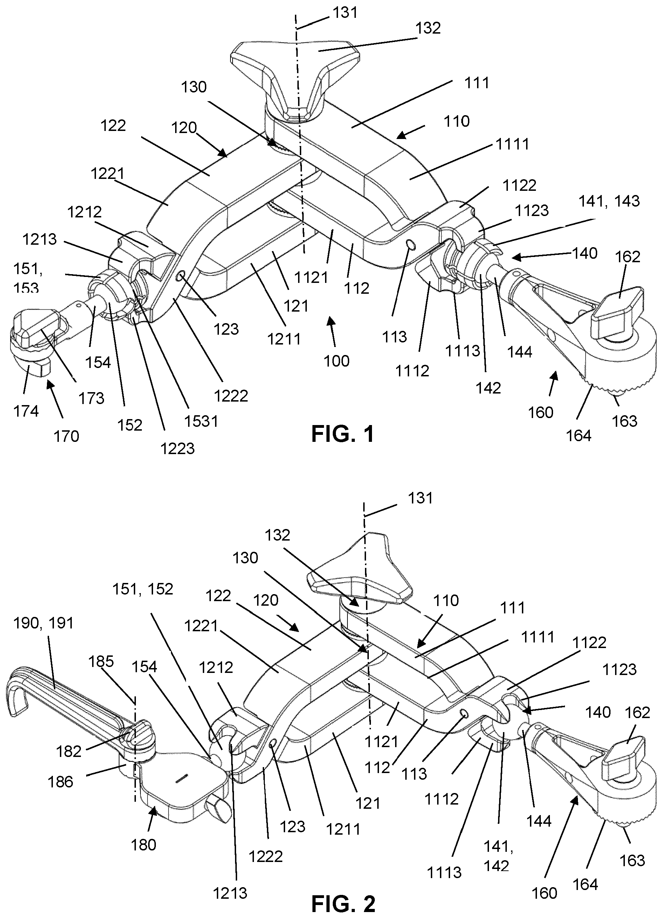

Ein in Fig. 1 gezeigte Positionierarm 100 weist ein erstes Armelement 110 und ein zweites Armelement 120 auf, die durch ein zentrales Gelenk 130 miteinander verbunden sind. An einem dem zentralen Gelenk 130 entfernten Ende weist das erste Armelement 110 ein weiteres Gelenk 140 auf, durch welches der Positionierarm 100 mit einer Befestigungsvorrichtung 160 verbindbar ist.

An einem dem zentralen Gelenk 130 entfernten Ende weist das zweite Armelement 120 ein weiteres Gelenk 150 auf, durch welches der Positionierarm 100 mit einem Universal-Adapter 170 verbindbar ist.

Das erste Armelement 110 wird gebildet von einem äußeren Zangenarm 111 und einem inneren Zangenarm 112, die an einem beispielsweise von einem Bolzen 1131 gebildeten Verbindungsgelenk 113 miteinander verbunden sind. Der äußere Zangenarm 111 weist ausgehend vom mittleren Gelenk 130 einen Hebelarm 1111 und zwischen dem Verbindungsgelenk 113 und dem weiteren Gelenk 140 einen Greifarm 1112 auf. Der innere Zangenarm 112 weist ausgehend vom mittleren Gelenk 130 einen Hebelarm 1121 und hinter dem Verbindungsgelenk 113 in Richtung zum weiteren Gelenk 140 einen Greifarm 1122 auf.

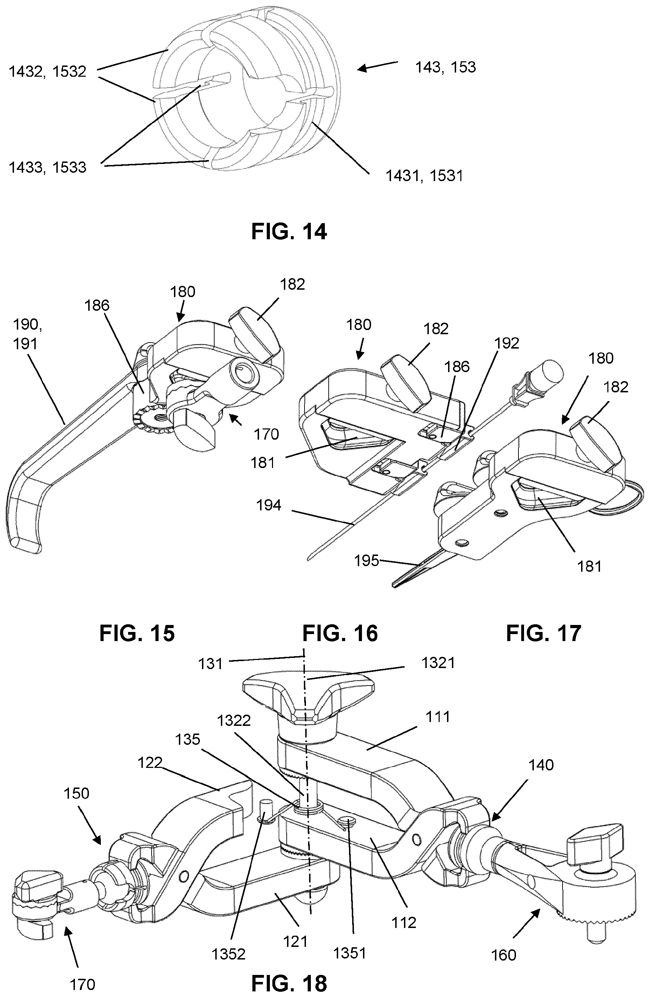

Am Greifarm 1112 ist wenigstens ein Greifer 1113 ausgebildet und am Greifarm 1122 ist wenigstens ein Greifer 1123 ausgebildet. Die Innenseite des Greifarms 1112 weist eine konkave Mulde 1114 und die Innenseite des Greifarms 1122 eine konkave Mulde 1124 auf. Die Greifer 1113 des Greifarm 1112 und die Greifer 1123 des Greifarms 1122 umschließen einen Klemmkörper 141 des weiteren Gelenks 140, der in Figur 1 in Form einer Drehhülse 143 ausgebildet ist. Die Drehhülse 143 ist in Figur 14 vergrößert dargestellt. Die Drehhülse 143 weist in einem Abstand von ihrem hinteren Ende eine Ringnut 1431 auf. Die Drehhülse ist durch vier achsparallel gleichmäßig im Außenmantel verteilt angeordnete Schlitze 1433 in vier Segmente 1432 unterteilt. Die Schlitze 1433 erstrecken sich über die Ringnut 1431 hinaus. Die vordere kreisförmige Öffnung der Drehhülse 143 ist vom Durchmesser so ausgelegt, dass eine Kugel 142 durch leichtes Aufweiten der Segmente 1432 in den Innenraum der der Drehhülse 143 einführbar ist.

Der Innenraum der Drehhülse 143 weist bevorzugt eine an die Kugel 142 angepasste bauchige Form auf.

Die Kugel 142 ist mit einem zylindrischen Anschlussstück 144 verbunden, an dem bevorzugt ein nicht dargestelltes Außengewinde ausgebildet ist, welches in ein passendes ebenfalls nicht dargestelltes Innengewinde der Befestigungsvorrichtung 160 einschraubbar ist.

Das zweite Armelement 120 wird gebildet von einem äußeren Zangenarm 121 und einem inneren Zangenarm 122, die in einem beispielsweise von einem Bolzen 1231 gebildeten Verbindungsgelenk 123 schwenkbar miteinander verbunden sind. Der äußere Zangenarm 121 weist ausgehend vom mittleren Gelenk 130 einen Hebelarm 1211 und zwischen dem Verbindungsgelenk 123 und dem weiteren Gelenk 150 einen Greifarm 1212 auf. Der innere Zangenarm 122 weist ausgehend vom mittleren Gelenk 130 einen Hebelarm 1222 und hinter dem Verbindungsgelenk 123 in Richtung zum weiteren Gelenk 150 einen Greifarm 1222 auf.

Am Greifarm 1212 ist wenigstens ein Greifer 1213 ausgebildet und am Greifarm 1222 ist wenigstens ein Greifer 1223 ausgebildet. Die Innenseite des Greifarms 1212 weist eine konkave Mulde 1214 und die Innenseite des Greifarms 1222 weist eine konkave Mulde 1224 auf. Die Greifer 1213 des Greifarms 1212 und die Greifer 1223 des Greifarms 1222 umschließen einen Klemmkörper 151 des weiteren Gelenks 150, der in Figur 1 in Form einer Drehhülse 153 gebildet ist. Die Drehhülse 153 ist in Figur 14 vergrößert dargestellt. Die Drehhülse 153 weist in einem Abstand von ihrem hinteren Ende eine Ringnut 1531 auf. Die Drehhülse 153 ist durch vier achsparallel gleichmäßig im Außenwandel verteilt angeordnete Schlitze 1533 in vier Segmente 1532 unterteilt. Die Schlitze 1533 erstrecken sich über die Ringnut 1531 hinaus. Die vordere kreisförmige Öffnung der Drehhülse 153 ist vom Durchmesser so ausgelegt, dass eine Kugel 152 durch leichtes Aufweiten der Segmente 1532 in den Innenraum der Drehhülse 153 einführbar ist. Der Innenraum der Drehhülse 153 weist bevorzugt eine an die Kugel 152 angepasste bauchige Form auf.

Die Kugel 152 ist mit einem zylindrischen Anschlussstück 154 verbunden, an dem bevorzugt einen nicht dargestelltes Außengewinde ausgebildet ist, welches in ein passendes, ebenfalls nicht dargestelltes Innengewinde eines Trägers 171 des Universal-Adapters 170 einschraubbar ist.

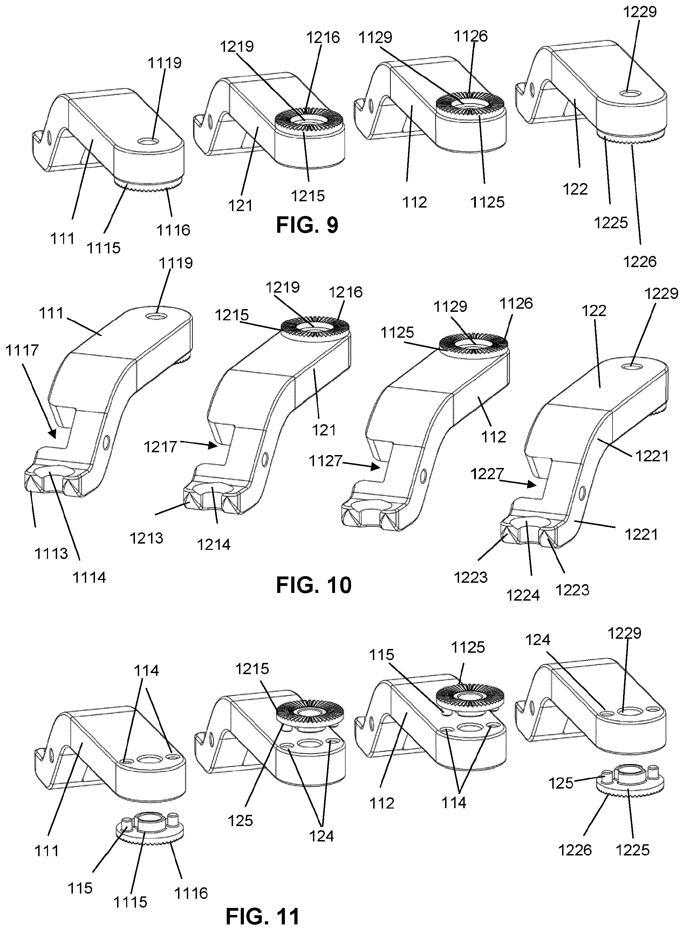

In den Figuren 9 bis 11 sind die vier Zangenarme 111, 112, 121 und 122 als Einzelteile dargestellt. Aus den Darstellungen ist ersichtlich, dass die vier Zangenarme 111, 112, 121 und 122 weitestgehend als Gleichteile herstellbar sind, wodurch die Herstellung und Lagerhaltung wesentlich erleichtert wird. Ferner ist aus diesen Figuren ersichtlich, dass der Zangenarm 111 an seinem dem mittleren Gelenk 130 zugewandten Ende eine als Durchgangsbohrung ausgebildete Bohrung 1119 aufweist. In gleicher Weise weist der Zangenarm 112 eine Bohrung 1129, der Zangenarm 121 eine Bohrung 1219 und der Zangenarm 122 eine Bohrung 1229 auf.

Wenigstens zwei der Zangenarme 111, 112, 121 und 122 weisen Rastelemente 1115, 1125, 1215 und 1225 auf. In den Figuren 9 und 10 sind die Rastelemente 1115, 1125, 1215 und 1225 unmittelbar an den Zangenarmen 111, 112, 121 und 122 ausgebildet. Die Rastelemente 1115, 1125, 1215 und 1225 weisen Verzahnungen 1116, 1126, 1216 und 1226 auf.

In einer in Figur 11 gezeigten alternativen Ausgestaltung sind die mit Verzahnungen 1116, 1126, 1216 und 1226 versehenen Rastelemente 1115, 1125, 1215 und 1225 als eigenständige scheibenförmige Bauteile ausgebildet und weisen auf der den Verzahnungen gegenüberliegenden Seite jeweils zwei Bolzen 115 auf, die mit entsprechenden Aufnahmebohrungen 114 an den Zangenarmen 111, 112, 121 und 122 formschlüssig in Eingriff bringbar sind.

Die Zangenarme 111, 112, 121 und 122 weisen eine abgekröpfte Form auf, wobei etwa in der Mitte der Kröpfung eine quer verlaufende Bohrung zur Bildung der Verbindungsgelenke 113 bzw. 123 mittels eines durchsteckbaren Bolzens vorgesehen ist. In diesen Bereichen der quer verlaufenden Bohrungen sind an den vier Zangenarmen 111, 112, 121 und 122 rechteckförmige Aussparungen 1117, 1127, 1217 und 1227 ausgebildet, die etwa bis zur Mitte der jeweiligen Zangenarme reichen und im montierten Zustand ein Ineinandergreifen jeweils eines Paares von Zangenarmen 111 mit 112 und 121 mit 122 ermöglichen.

Die Zangenarme 111, 112, 121 und 122 lassen sich als Gleichteile in robuster Ausführungsform als Aluminiumdruckgussteil oder als Kunststoffspritzgussteil kostengünstig herstellen.

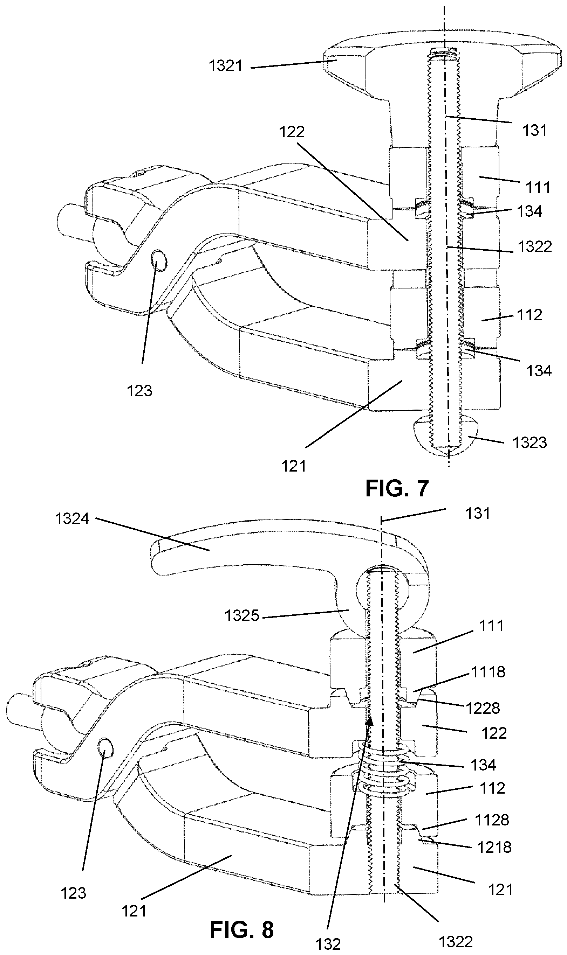

Das mittlere Gelenk 130 bildet eine Schwenkachse 131 für das erste Armelement 110 und das zweite Armelement 120. Das zentrale oder mittlere Gelenk 130 verbindet - wie am besten aus Figur 7 ersichtlich - mittels einer Gewindestange 1322 durch die Bohrungen 1119, 1129, 1219 und 1229 hindurch die hinteren Enden des vom äußeren Zangenarm 111 und vom inneren Zangenarm 112 zangenförmig gebildeten ersten Armelements 110 und die hinteren Enden des vom äußeren Zangenarm 121 und vom inneren Zangenarm 122 zangenartig gebildeten zweiten Armelements 120.

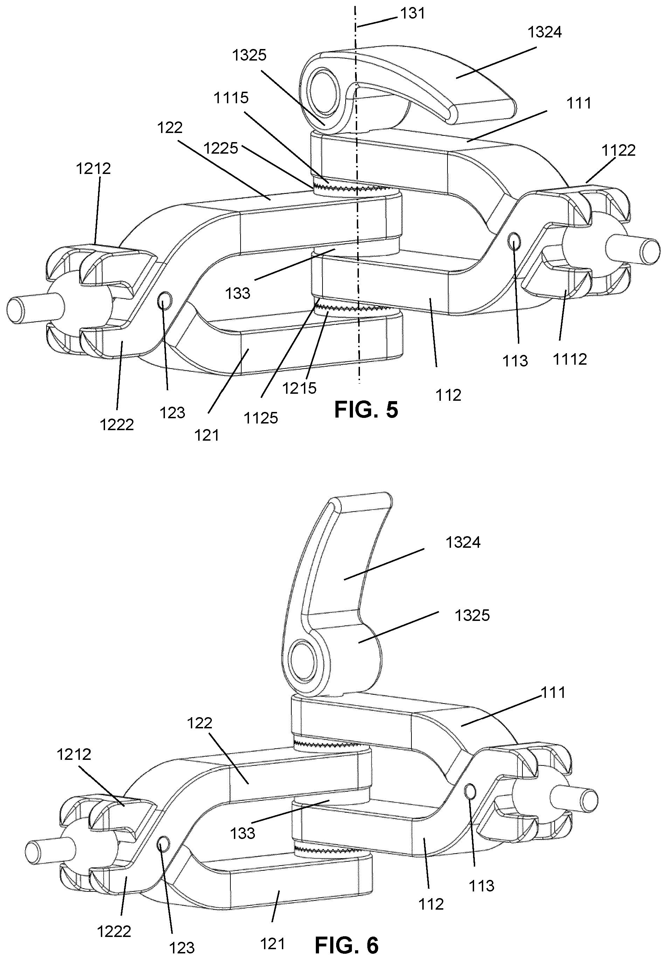

Dabei liegen jeweils abwechselnd von unten nach oben der äußere Zangenarm 121 des zweiten Armelements 120, der innere Zangenarm 112 des ersten Armelement 110, der innere Zangenarm 122 des zweiten Armelement 120 und der äußere Zangenarm 111 des ersten Armelements 110 übereinander. Die Gewindestange 1322 ist an ihrem oberen Ende mit einem Bedienelement verbunden, das als Drehgriff 1321 oder - wie in den Figuren 5, 6 und 8 dargestellt - als mit einem Exzenter 1325 versehener Spanngriff 1324 ausgebildet ist.

Das untere Ende der Gewindestange 1322 ist entweder - wie in Figur 7 dargestellt - in eine Mutter 1323 oder - wie in Figur 8 dargestellt - unmittelbar in ein im Bereich der Bohrung 1219 ausgebildetes Innengewinde des äußeren Zangenarms 121 eingeschraubt.

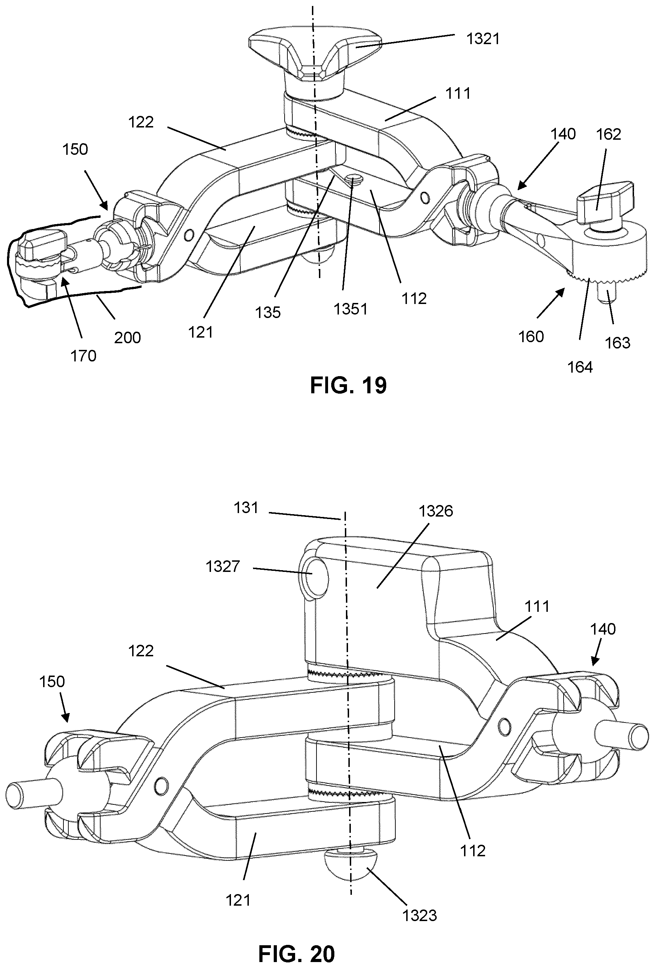

Die Gewindestange 1322 bildet in Verbindung mit den Bedienelementen, nämlich dem Drehgriff 1321, dem Spanngriff 1324 oder einem in Figur 20 angedeuteten Motor 1326 und der Verankerung in der Mutter 1323 oder dem Innengewinde des Zangenarms 121 eine Blockiereinrichtung 132.

Der Motor 1326 wird bevorzugt durch eine in dem vorzugsweise integral am Zangenarm 111 ausgebildeten Gehäuse angeordneten Akku angetrieben, wobei die Bedienelemente 1327 ergonomisch günstig angeordnet sind und das Gehäuse, wie in Figur 20 dargestellt, ebenfalls ergonomisch günstig als Griffstück beim Führen des Positionierarms 100 in die gewünschte Position verwendbar ist.

Ein Anziehen der Bedienelemente 1321, 1324 bzw. 1326 presst die Enden der Zangenarme 111, 112, 121 und 122 gegeneinander. In einem nur leicht angezogenen Zustand ist noch eine Schwenkbewegung um die Schwenkachse 131 möglich. Bei einem starken Anziehen der Bedienelemente 1321, 1324 bzw. 1326 wird dagegen die Schwenkbewegung der Armelement 110 und 120 relativ zueinander im zentralen Gelenk 130 blockiert.

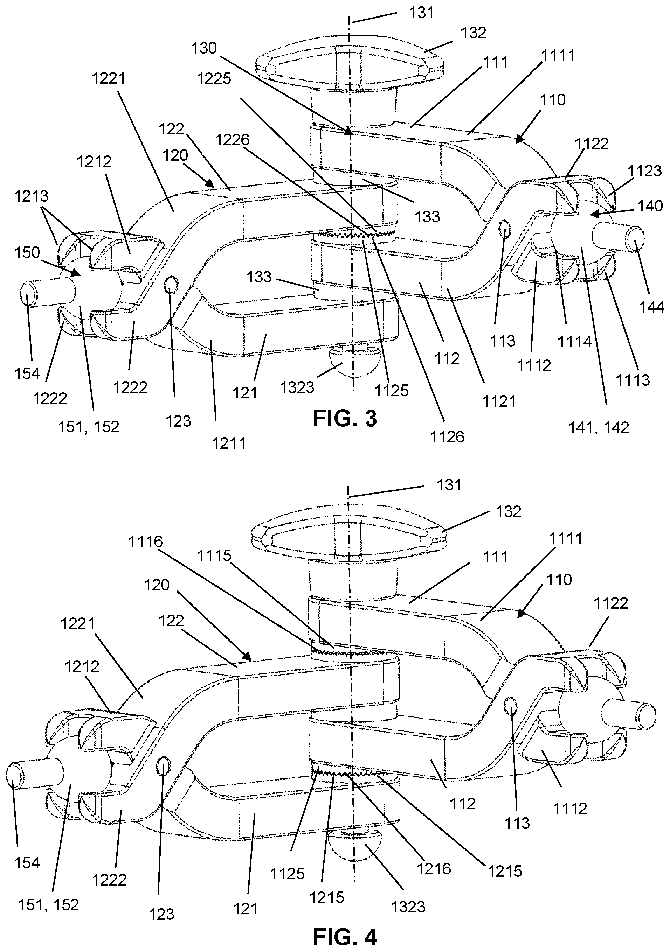

Dieses Blockieren kann durch die ineinandergreifenden Verzahnungen 1116, 1126, 1216 und 1226 der Rastelemente 1115, 1125, 1215 und 1225 verstärkt werden. Hierbei sind zwei Varianten möglich. Wie in Figur 3 gezeigt ist in einer ersten Variante ein zentraler Eingriff der beiden Rastelemente 1126 und 1226 möglich. Diese Ausführungsform wird dann bevorzugt verwendet, wenn eine Blockierung der weiteren Gelenke 140 und 150 vor einem Blockieren des mittleren Gelenks 130 erwünscht ist.

Zum anderen ist - wie in Figur 4, 5, 6 und 7 dargestellt - in einer zweiten Variante auch ein dezentraler Eingriff an zwei Paaren von Rastelementen 1115 mit 1225 und 1215 mit 1125 möglich. In diesem Falle ist eine Zwischenlage einer Distanzhülse 133 von Vorteil. Diese zweite Variante wird bevorzugt verwendet, wenn eine Blockierung des mittleren Gelenks 130 vor einem Blockieren der weiteren Gelenke 140 und 150 erwünscht ist.

Durch das Blockieren des mittleren Gelenks 130 mittels der Blockiereinrichtung 132 werden gleichzeitig oder geringfügig zeitversetzt auch die weiteren Gelenke 140 bzw. 150 blockiert. Beim Zusammendrücken der Zangenenden an den Hebelarmen 1111, 1121, 1211 und 1221 der Zangenarme 111, 112, 121 und 122 werden gleichzeitig die Greifarme 1112 und 1122 des ersten Armelements 110 und die Greifarme 1212 und 1222 des zweiten Armelements 120 zusammengedrückt. Sie umschließen dabei die Klemmkörper 141 bzw. 151, die entweder von Kugeln 142 bzw. 152 oder von Drehhülsen 143 bzw. 153 gebildet sind, wobei die Drehhülsen 143 bzw. 153 ihrerseits gemäß einer besonders vorteilhaften Variante auch zur Aufnahme von Kugeln 142 bzw. 152 geeignet sind.

Durch unterschiedliche Durchmesser der Kugeln 142 bzw. 152 und/oder durch unterschiedlich starke Distanzhülsen 133 lässt sich sehr genau einstellen, welches der Gelenke 130, 140 und oder 150 beim Betätigen der Blockiereinrichtung 132 zuerst blockiert und welches zuletzt blockiert. Gemäß einer vorteilhaften Variante kann dabei beispielsweise zuerst das Gelenk 140 blockieren, nachdem der Positionierarm 100 grob in die benötigte Gebrauchsposition geschwenkt wurde. Beim weiteren zuziehen der Blockiereinrichtung 132 kann dann beispielsweise das zentrale Gelenk 130 als nächstes blockieren und als letztes das weitere Gelenk 150, nachdem dort ein am Universal-Adapter 170 angeordnetes Instrument 190 in die Zielposition gedreht bzw. geschwenkt worden ist. Die gerade beschriebene Reihenfolge ist nur exemplarisch zu sehen. Es ist auch jede andere Reihenfolge mit einer entsprechenden Abstimmung der Durchmesser oder der Durchmesser der Kugeln 142 bzw. 152 und/oder der Drehhülsen 143 bzw. 153 und/oder der Dicke der Distanzscheiben 133 möglich. Auch über eine Veränderung der Längenverhältnisse der Hebelarme zu den Greifarmen am jeweiligen Zangenarm-Paar kann auf den Zeitpunkt des Blockierens des jeweiligen Gelenks 130, 140 bzw. 150 Einfluss genommen werden.

Anstelle der Rastelemente 1115, 1125, 1215 und 1225 können auch - wie in Figur 8 gezeigt - ein in einen Innenkonus 1228 am Zangenarm 122 eingreifender Außen-Konus 1118 am Zangenarm 111 und ein in einen Innenkonus 1128 am Zangenarm 112 eingreifender Außenkonus 1218 am Zangenarm 121 die Blockiereinrichtung 132 beim Blockieren des mittleren zentralen Gelenks 130 unterstützen.

Damit sich die Blockiereinrichtung 132 des zentralen Gelenks 130 beim Öffnen des Drehgriff 1321, des Spanngriffs 1324 oder des Motors 1326 leicht öffnet, ist es vorteilhaft, wenn wenigstens ein Federelement 134 der Schließkraft entgegenwirkt. In Figur 8 ist zu diesem Zweck im mittleren Bereich der Gewindestange 1322 als axiales Federelement 134 eine die Gewindestange 1322 umgebende Spiral-Druckfeder angeordnet. In Figur 7 sind zwei als Tellerfedern ausgebildete Federelemente 134 axial beabstandet voneinander zwischen den Zangenarmen 111 und 122 bzw. zwischen den Zangenarmen 112 und 121 die Gewindestange 1322 umgebend angeordnet.

Die Handhabung des erfindungsgemäßen Positionierarms 100 wird vorteilhaft zusätzlich dadurch erleichtert, dass, wie in den Figuren 18 und 19 dargestellt, ein weiteres Federelement 135 vorgesehen ist, mittels dem das erste Armelement 110 und das zweite Armelement 120 in einer definierte Position zueinander federnd vorbelastet sind. Zweckmäßigerweise ist dies eine gestreckte Position, bei der das erste Armelement 110 und das zweite Armelement 120 einen Winkel von 180° einschließen. Das weitere Federelement 135 ist im gezeigten Ausführungsbeispiel als Drehschenkelfeder ausgebildet, die die Gewindestange 1322 umgibt und deren Enden an einem Anschlag 1351 am Zangenarm 112 bzw. an einem Anschlag 1352 am Zangenarm 122 festgelegt sind.

Die Befestigungsvorrichtungen 160 ist, wie aus Figur 19 ersichtlich, mittels einer mit einem Drehgriff 162 verbundenen Schraube 163 in einen nicht dargestellten Halter einschraubbar, der beispielsweise an seitlichen Schienen eines Operationstisches befestigbar ist. Rastverzahnungen 164 können auch hier das sichere Fixieren des Positionierarms 100 unterstützen.

Am patientenseitigen anderen Ende ist an das zweite Gelenk 150 anschließend besonders bevorzugt ein Universaladapter 170 vorgesehen, der beispielsweise durch Einschrauben in einen Träger 171 mit dem Anschlussstück 154 verbindbar ist. Der Universal-Adapter 170 weist einen Formkörper 173 auf, der besonders bevorzugt nach Art eines dreiseitigen Prismas ausgebildet ist. Der Formkörper 173 ist formschlüssig mit einer Aufnahme 181 an einem Halter 180 verbindbar, wobei der Formkörper 173 dabei keilförmig gegen die zwei schrägen vorderen Flanken der Aufnahme 181 gedrückt wird, wenn mittels eines Drehgriffs 182 ein am Halter 180 verschiebbar gelagerter Schieber 184 von hinten gegen ihn gedrückt wird. Der Universaladapter 170 kann zusätzlich eine Drehachse 172 aufweisen, die von einer Schraube 175 mit einem daran angeordneten Drehgriff 174 gebildet wird (Figur 19). Rastscheiben 176 am Träger 171 und Rastscheiben 177 am Formkörper 173 ermöglichen eine fein drehbare Rasterung und Arretierung des Formkörpers 173 gegenüber dem Träger 171, sodass der auf dem Formkörper 173 sitzende Halter 180 mit den Werkzeugen 190 entsprechend fein drehbar und arretierbar ist.

Das Prisma des Formkörpers 173 hat keine scharfen Ecken und Kanten. Wenn man eine sterile Abdeckfolie 200 (siehe Fig. 19) dazwischen legt, wird die Folie 200 nicht verletzt. Dadurch wird gewährleistet, dass die sterile Barriere, die durch die Abdeckfolie 200 gebildet wird, nicht verletzt wird. Gleichzeitig erlaubt das Prisma des Formkörpers 173 immer einen guten und vor allem sicheren Formschluss (mit und ohne dazwischen liegender Abdeckfolie 200), der gewährleistet dass die Position des am Universaladapter 170 angebrachten Instruments 190 nicht verändert wird.

Wie aus den Figuren 15 bis 17 ersichtlich, lassen sich mit dem Universal-Adapter 170 unterschiedlichste Werkzeuge und Instrumente 190 einfach und schnell verbinden. In Figur 15 wird das Werkzeug 190 von einem Haken 191 gebildet. In Figur 16 ist am Halter 180 ein Werkzeughalter 186 angeordnet, an dem im gezeigten Ausführungsbeispiel eine Nadelführung 192 zur Führung einer Nadel 194 gelagert ist. In Figur 17 ist der Halter 180 beispielhaft mit einer durch Festklemmen am Halter 180 fixierbaren Pinzette 195 bestückt. Der Halter 180 ist bevorzugt mit einer weiteren Drehachse 185 versehen, sodass das Werkzeug 190 gegenüber dem Werkzeughalter 186 drehbar und mittels eines Drehgriffs 187 arretierbar ist.

Der Universal-Adapter 170 stellt für sich gesehen ein eigenständig erfinderisches Merkmal dar, da dieser mit seinem Formkörper 173 mit beliebigen Haltern 180 für beliebige Werkzeuge 190, 191, 192, 193, 194 und 195 verbunden werden kann. Dadurch können beliebige Werkzeuge 190, 191, 192, 193, 194 und 195 je nach Erfordernis während einer Operation an mehreren derartigen Positionierarmen 100 beliebig platziert und schnell gewechselt werden. Der erfindungsgemäße Positionierarm 100 ist auch zur Anbringung von bevorzugt fernbetätigbaren Robotersystemen oder von Patientenlagerungssystemen am Universal-Adapter 170 geeignet. Die Verriegelung der Gelenke 130, 140, 150 ist in blockiertem Zustand derart stabil, das am Universal-Adapter 170 der gesamt OP-Tisch in die Höhe gehoben werden kann, ohne dass sich die dabei die eingestellte Position der Gelenke 130, 140, 150 auch nur im geringsten verändert.

Wie aus den Figuren 9 bis 11 ersichtlich, besteht der erfindungsgemäße Positionierarm 100 aus sehr wenigen leicht demontierbaren und montierbaren Zangenarmen 111, 112, 121 und 122, so dass ein Zerlegen zum Sterilisieren in einfacher Weise möglich ist.

Die Sterilität lässt sich auch in einfacher Weise dadurch erzeugen, dass über den gesamten Positionierarm 100 eine sterile Abdeckfolie als Hülle 200 gezogen wird. Dies ist in Figur 19 im Bereich des weiteren Gelenks 150 nur angedeutet. Der Universal-Adapter 170 ermöglicht ein zerstörungsfreies Einklemmen dieser Hülle 200 zwischen dem Formkörper 173 und der Aufnahme 181 am Halter 180.

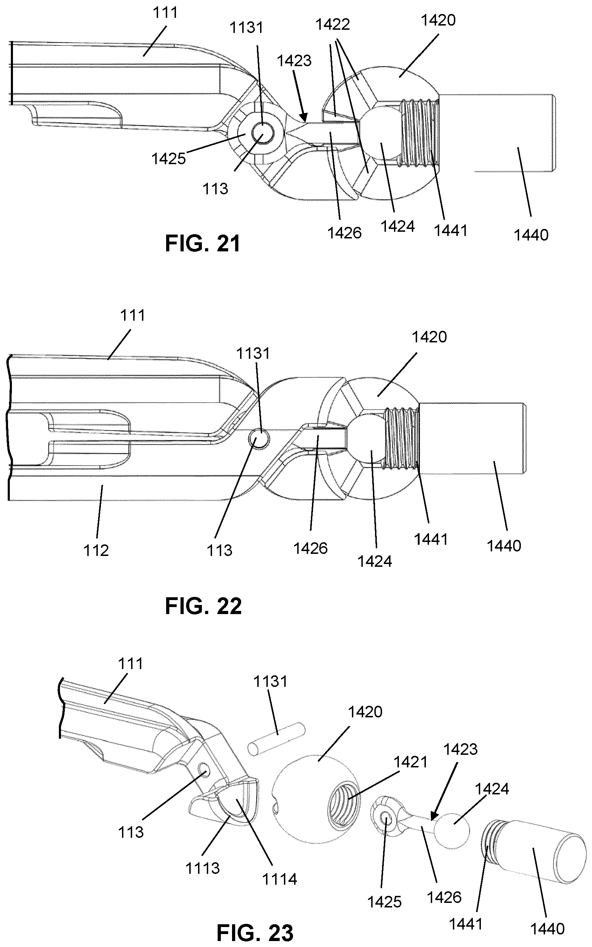

In den Figuren 21 bis 24 ist eine weitere Variante eines Zangenarms dargestellt. Bei dieser wird der Klemmkörper von einer teilweise hohlen Kugel 1420 gebildet, In einer Bohrung der Kugel 1420, deren eines Ende mit einem Gewinde 1421 und deren anderes Ende mit kreuzförmig zueinander angeordneten Schlitzen 1422 versehen ist, ist das eine Ende einer Zugstange 1423 mit einem Kugelkopf 1424 gelagert. Ein Schaft 1426 der Zugstange 1423 durchdringt die Schlitze 1422 und die Zugstange 1423 wird mit einem dem Kugelkopf 1424 gegenüberliegenden, am anderen Ende der Zugstange 1423 befindlichen Lagerauge 1425 am Verbindungsgelenk 113 bzw. einem das Verbindungsgelenk bildenden Bolzen 1131 gelagert.

Nach Einführen der Zugstange 1423 in die Bohrung der Kugel 1420 wird ein Anschlussstück 1440 mit seinem an einem Ende angebrachten Gewinde 1441 in das Gewinde 1421 der Kugel 1420 eingeschraubt.

Die Kugel 1420 wird mittels der Zugstange 1423 beim Zusammendrücken der Zangenarme 111 und 112 gegen eine in diesem Fall flachere Mulde 1114 eines Greifers 1113 am Ende der Zangenarme 111 und 112 gezogen und dadurch eine Bewegung der Kugel 1420 arretiert. Die Zähne des Greifers 1113 sind bei dieser Variante kürzer ausgebildet, als bei den zuerst beschriebenen Varianten, bei denen die Zähne der Greifer 1113, 1123, 1213, 1223 die Klemmkörper 141, 151 bzw. die Kugeln 142, 152 zu mehr als der Hälfte von deren Durchmesser umgreifen.

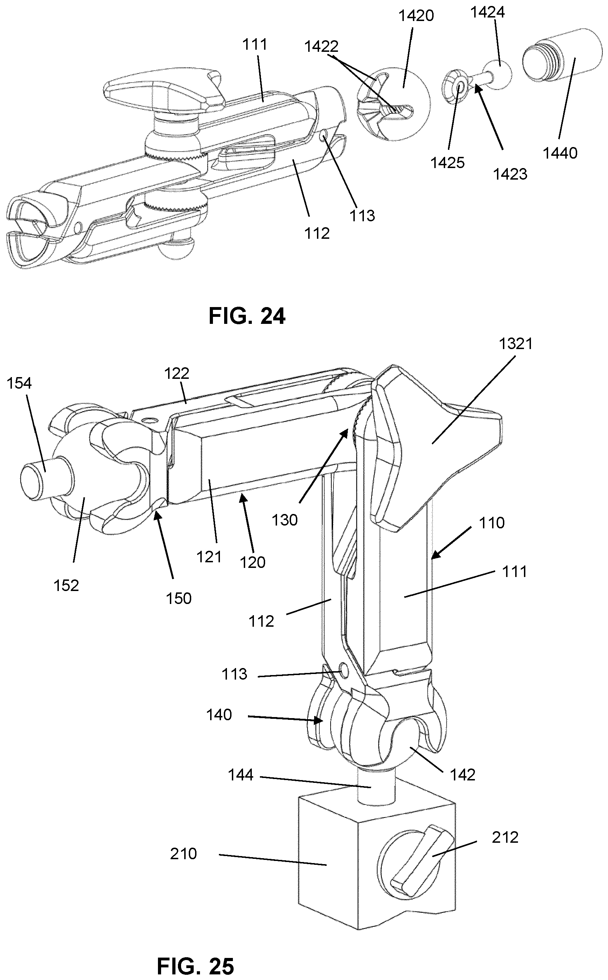

In Figur 25 ist eine weitere Befestigungsmöglichkeit eines erfindungsgemäßen Positionierarms dargestellt, bei der ein Anschlussstück 144 eines Gelenks 140 mit einem Magnethalter 210 verbunden ist, der mittels eines Magnetschalters 212 in eine Wirkstellung oder außer Betrieb gesetzt werden kann. Mittels des Magnethalter 210 kann dieser Positionierarm sehr einfach auf metallischen Flächen fixiert und wieder gelöst werden.

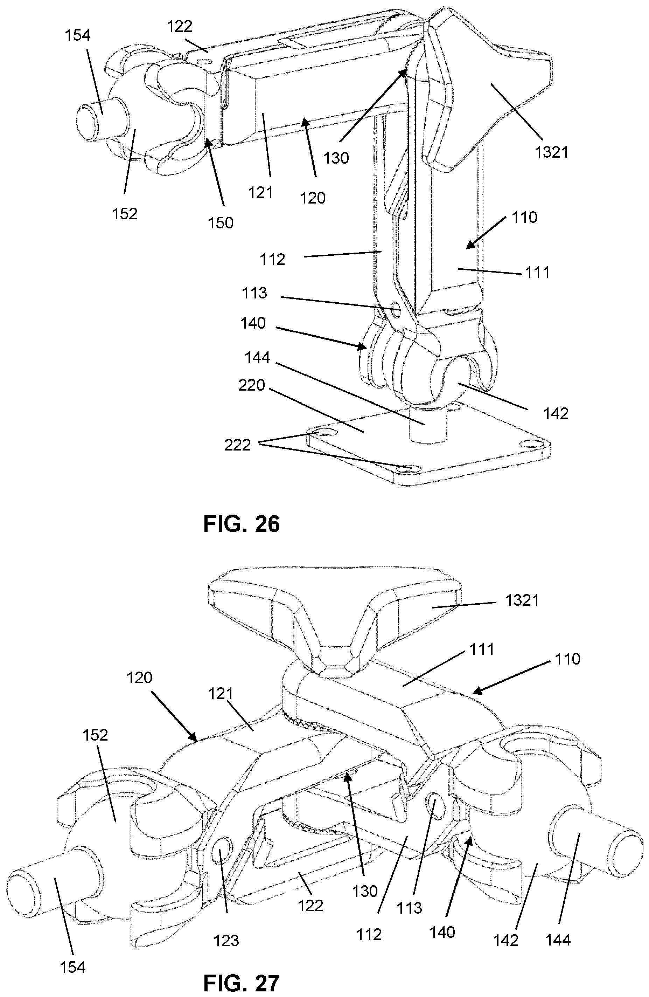

In Figur 26 ist ein Anschlussstück 144 eines Gelenks 140 mit einer Flanschplatte 220 verbunden, die mehrere Bohrungen 222 zur Befestigung beispielsweise mittels Schrauben an einem Träger oder an einer Wand aufweist.

In Figur 27 ist eine besonders kleine Ausführungsform eines Positionierarms gezeigt, bei der die Zangenarme 111, 112; 121 und 122 sehr kurz und kompakt ausgeführt sind.

### BEZUGSZEICHENLISTE

- 100: Positionierarm

- 110: (erstes) Armelement
- 111: (äußerer) Zangenarm (von 110)
- 1111: Hebelarm (von 111)
- 1112: Greifarm (von 111)
- 1113: Greifer
- 1114: Mulde
- 1115: Rastelement
- 1116: Verzahnung
- 1117: Aussparung
- 1118: (Außen-) Konus
- 1119: Bohrung
- 112: (innerer) Zangenarm (von 110)
- 1121: Hebelarm (von 112)
- 1122: Greifarm (von 112)
- 1123: Greifer
- 1124: Mulde
- 1125: Rastelement
- 1126: Verzahnung
- 1127: Aussparung
- 1128: (Innen-) Konus
- 1129: Bohrung
- 113: Verbindungsgelenk (von 111 und 112)
- 1131: Bolzen
- 114: Aufnahmebohrung
- 115: Bolzen

- 120: (zweites) Armelement
- 121: (äußerer) Zangenarm (von 120)
- 1211: Hebelarm (von 121)
- 1212: Greifarm (von 121)
- 1213: Greifer
- 1214: Mulde
- 1215: Rastelement
- 1216: Verzahnung
- 1217: Aussparung
- 1218: (Außen-) Konus
- 1219: Bohrung
- 122: (innerer) Zangenarm (von 120)
- 1221: Hebelarm (von 122)
- 1222: Greifarm (von 122)
- 1223: Greifer
- 1224: Mulde
- 1225: Rastelement
- 1226: Verzahnung
- 1227: Aussparung
- 1228: (Innen-) Konus
- 1229: Bohrung
- 123: Verbindungsgelenk (von 121 und 122)

- 130: (zentrales) Gelenk
- 131: Schwenkachse
- 132: Blockiereinrichtung
- 1321: Drehgriff
- 1322: Gewindestange
- 1323: Mutter
- 1324: Spanngriff
- 1325: Exzenter
- 1326: Motor
- 1327: Bedienelement
- 133: Distanzscheibe
- 134: (axiales) Federelement
- 135: Federelement (Schenkelfeder)

- 140: (weiteres) Gelenk
- 141: Klemmkörper
- 142: Kugel
- 1420: Kugel (in Figuren 21 bis 24)
- 1421: Gewinde (in 1420)
- 1422: Schlitz (in 1420)
- 1423: Zugstange
- 1424: Kugelkopf (an 1423)
- 1425: Lagerauge (an 1423)
- 1426: Schaft (von 1423)
- 143: Drehhülse
- 1431: Ringnut
- 1432: Segment
- 1433: Schlitz
- 144: Anschlussstück
- 1440: Anschlussstück (in Figuren 21 bis 24)
- 1441: Gewinde (an 1440)

- 150: (weiteres) Gelenk
- 151: Klemmkörper
- 152: Kugel
- 153: Drehhülse
- 1531: Ringnut
- 1532: Segment
- 1533: Schlitz
- 154: Anschlussstück
- 160: Befestigungsvorrichtung
- 162: Drehgriff
- 163: Schraube
- 164: Rastverzahnung

- 170: (Universal-)Adapter
- 171: Träger
- 172: Drehachse
- 173: Formkörper
- 174: Drehgriff
- 175: Schraube
- 176: Rastscheibe (an 171)
- 177: Rastscheibe (an 173)

- 180: Halter
- 181: Aufnahme
- 182: Drehgriff
- 183: Schraube
- 184: Schieber
- 185: Drehachse
- 186: Werkzeughalter
- 187: Drehgriff

- 190: Werkzeug, Instrument
- 191: Haken
- 192: Nadelführung
- 193: Werkzeugträger
- 194: Nadel
- 195: Pinzette
- 200: (sterile) Abdeckfolie (Hülle)

- 210: Magnethalter
- 212: Magnetschalter

- 220: Flanschplatte
- 222: Bohrung (in 220)

## Patentansprüche

1. Positionierarm (100) zum Positionieren von Instrumenten, Geräten oder Werkzeugen,
wobei der Positionierarm (100) mindestens ein erstes Armelement (110) und ein zweites Armelement (120) aufweist, die mittels eines zentralen Gelenks (130) um eine Schwenkachse (131) schwenkbar miteinander verbunden sind,
wobei zumindest eines der Armelemente (110, 120) ein weiteres Gelenk (140, 150) an einem dem zentralen Gelenk (130) entgegengesetzten Ende aufweist,
wobei der Positionierarm (100) eine Blockiereinrichtung (132) zum Blockieren und Freigeben des zentralen Gelenks (130) und des zumindest einen weiteren Gelenks (140, 150) aufweist,
wobei die Blockiereinrichtung (132) am zentralen Gelenk (130) angeordnet ist und über zumindest eine an den Armelementen (110, 120) ausgebildete Übertragungseinrichtung (111, 112; 121, 122) das Blockieren und Freigeben der weiteren Gelenke (140, 150) bewirkt,
wobei die Übertragungseinrichtung (111, 112; 121, 122) von einem Paar von äußeren und inneren Zangenarmen (111, 112; 121, 122) gebildet wird, die gleichzeitig auch die Armelemente (110, 120) bilden,
wobei die äußeren und inneren Zangenarme (111, 112; 121, 122) jeweils einen Hebelarm (1111, 1121; 1211, 1221) und einen Greifarm (1112, 1122; 1212, 1222) aufweisen,
wobei die Hebelarme (1111, 1121; 1211, 1221) mit ihren hinteren Enden fluchtend im Bereich des zentralen Gelenks (130) mittels der Blockiereinrichtung (132) verbindbar sind, so dass der äußere Zangenarm (121) des zweiten Armelements (120), der innere Zangenarm (112) des ersten Armelements (110), der innere Zangenarm (122) des zweiten Armelements (120) und der äußere Zangenarm (111) des ersten Armelements (110) abwechselnd übereinander liegen, und
wobei die Greifarme (1112, 1122; 1212, 1222) einen Teil der weiteren Gelenke (140, 150) bilden, und
**dadurch gekennzeichnet, dass** beim Zusammendrücken der Enden an den Hebelarmen (1111, 1121, 1211 und 1221) gleichzeitig die Greifarme (1112, 1122; 1212, 1222) zusammengedrückt werden.

2. Positionierarm (100) nach Anspruch 1,
wobei beide Armelemente (110, 120) jeweils ein weiteres Gelenk (140; 150) aufweisen, und wobei die Blockiereinrichtung (132) jeweils eine Übertragungseinrichtung (111, 112; 121, 122) zu dem weiteren Gelenk (140; 150) aufweist.

3. Positionierarm (100) nach Anspruch 1 oder 2,
wobei das weitere Gelenk (140; 150) einen drehbaren Klemmkörper (141; 151) aufweist, der vorzugsweise als Kugel (142; 152) und/oder als Drehhülse (143; 153) ausgebildet ist.

4. Positionierarm (100) nach einem der vorhergehenden Ansprüche,
wobei die Blockiereinrichtung (132) ein in axialer Richtung des zentralen Gelenks (130) verlagerbares, mit einer Betätigungsvorrichtung (1321, 1324, 1326) verbundenes Betätigungselement (1322) aufweist.

5. Positionierarm (100) nach Anspruch 4,
wobei die Betätigungsvorrichtung von einem Drehgriff (1321), von einem mit einem Exzenter (1325) verbundenen Spanngriff (1324) oder von einem Motor (1326) gebildet wird.

6. Positionierarm (100) nach einem der vorhergehenden Ansprüche,
wobei eines der weiteren Gelenke (140) mit einer zur Anbringung an einem Operationstisch geeigneten Befestigungsvorrichtung (160) versehen und ein anderes der weiteren Gelenke (150) zur Verbindung mit einem zur Befestigung unterschiedlicher Instrumente, Geräte oder Werkzeuge (190; 191; 192; 193) geeigneten Adapter (170) ausgebildet ist.

7. Positionierarm (100) nach einem der vorhergehenden Ansprüche, wobei die Zangenarme (111, 112; 121, 122) als Gleichteile gebildet sind.

8. Positionierarm (100) nach einem der vorhergehenden Ansprüche, wobei die Zangenarme (111, 112; 121, 122) aus einem Leichtmetall, einer Leichtmetall-Legierung oder einem Kunststoff-Material gebildet sind.

9. Positionierarm (100) nach einem der vorhergehenden Ansprüche, wobei die Blockiereinrichtung (132) an der Übertragungseinrichtung (111, 112; 121, 122) angeordnete oder ausgebildete Rastelemente (1125, 1225) aufweist.

10. Positionierarm (100) nach einem der Ansprüche 6 bis 9,
wobei der Adapter (170) wenigstens einen Formkörper (173) für ein Zusammenwirken mit einer komplementär geformten Aufnahme (181) eines Halters (180) für das Werkzeug, Gerät bzw. Instrument (190; 191; 192; 193) aufweist.

11. Positionierarm (100) nach Anspruch 10,
wobei der Formkörper (173) mittels eines am Halter (180) angeordneten Schiebers (184) in der Aufnahme (181) fixierbar ist.

12. Positionierarm (100) nach Anspruch 3, wobei die Blockiereinrichtung (132). für ein zeitversetztes Arretieren des zentralen Gelenks (130) und der weiteren Gelenke (140; 150) geeignet und eingerichtet ist, und zwar mittels einer entsprechenden Abstimmung und Einstellung der Betätigungswege der Blockiereinrichtung (132), der Übertragungseinrichtungen (111, 112; 121, 122) und des drehbaren Klemmkörper (141; 151).

13. Positionierarm (100) nach einem der vorhergehenden Ansprüche, wobei im Bereich des zentralen Gelenks (130) wenigstens ein der Schließkraft der Blockiereinrichtung (132) entgegen wirkendes Federelement (134) angeordnet ist.

14. Positionierarm (100) nach einem der vorhergehenden Ansprüche, wobei im Bereich des zentralen Gelenks (130) wenigstens ein zumindest eines der Armelemente (110; 120) in eine vorbestimmte Grundposition vorbelastendes Federelement (135) angeordnet ist.

15. Positionierarm (100) nach einem der Ansprüche 3 bis 14, wobei der Klemmkörper (141; 151) mittels einer Zugstange mit einem zwei Zangenarme (111, 112; 121, 122) verbindenden Verbindungsgelenk (113; 123) verbunden ist.

## Claims

1. Positioning arm (100) for positioning instruments, devices or tools,
wherein the positioning arm (100) comprises at least a first arm element (110) and a second arm element (120) which are pivotably connected to one another about a pivot axis (131) by means of a central joint (130),
wherein at least one of the arm elements (110, 120) comprises a further joint (140, 150) at an end opposite to the central joint (130),
wherein the positioning arm (100) comprises a blocking device (132) for blocking and releasing the central joint (130) and the at least one further joint (140, 150),
wherein the blocking device (132) is arranged at the central joint (130) and effects the blocking and unblocking of the further joints (140, 150) via at least one transmission device (111, 112; 121, 122) formed at the arm elements (110, 120),
wherein the transmission device (111, 112; 121, 122) is formed by a pair of outer and inner pincer arms (111, 112; 121, 122) which simultaneously also form the arm elements (110, 120),
wherein the outer and inner pincer arms (111, 112; 121, 122) each comprise a lever arm (1111, 1121; 1211, 1221) and a gripping arm (1112, 1122; 1212, 1222),
wherein the lever arms (1111, 1121; 1211, 1221) are configured to be connected with their rear ends aligned in the region of the central joint (130) by means of the blocking device (132), so that the outer pincer arm (121) of the second arm element (120), the inner pincer arm (112) of the first arm element (110), the inner pincer arm (122) of the second arm element (120) and the outer pincer arm (111) of the first arm element (110) lie alternately one above the other, and
wherein the gripping arms (1112, 1122; 1212, 1222) form a part of the further joints (140, 150),
**characterized in that**, when the ends of the lever arms (1111, 1121, 1211 and 1221) are pressed together, the gripping arms (1112, 1122; 1212, 1222) are simultaneously pressed together.

2. The positioning arm (100) according to claim 1, wherein both arm elements (110, 120) each comprise a further joint (140; 150) and wherein the blocking device (132) comprises a respective transmission device (111, 112; 121, 122) to the further joint (140; 150).

3. The positioning arm (100) according to claim 1 or 2, wherein the further joint (140; 150) comprises a rotatable clamping body (141; 151), which is preferably formed as a ball (142; 152) and/or as a rotating sleeve (143; 153).

4. The positioning arm (100) according to any one of the preceding claims, wherein the blocking device (132) comprises an actuating element (1322) displaceable in the axial direction of the central joint (130) and connected to an actuating device (1321, 1324, 1326).

5. The positioning arm (100) according to claim 4, wherein the actuating device is formed by a rotary handle (1321), by a clamping handle (1324) connected to an eccentric (1325) or by a motor (1326).

6. The positioning arm (100) according to any one of the preceding claims, wherein one of the further joints (140) is provided with a fastening device (160) suitable for attachment to an operating table, and another of the further joints (150) is designed for connection to an adapter (170) suitable for attaching different instruments, devices or tools (190; 191; 192; 193).

7. The positioning arm (100) according to any one of the preceding claims, wherein the pincer arms (111, 112; 121, 122) are configured as identical parts.

8. The positioning arm (100) according to any one of the preceding claims, wherein the pincer arms (111, 112; 121, 122) are made of a light metal, a light metal alloy or a plastic material.

9. The positioning arm (100) according to any one of the preceding claims, wherein the blocking device (132) comprises latching elements (1125, 1225) arranged or formed on the transmission device (111, 112; 121, 122).

10. The positioning arm (100) according to any one of claims 6 to 9, wherein the adapter (170) comprises at least one shaped body (173) for cooperation with a complementarily shaped receptacle (181) of a holder (180) for the tool, device or instrument (190; 191; 192; 193).

11. The positioning arm (100) according to claim 10, wherein the shaped body (173) is configured to be fixed in the receptacle (181) by means of a slider (184) arranged on the holder (180).

12. The positioning arm (100) according to claim 3, wherein the blocking device (132) is suitable and arranged for a time-delayed locking of the central joint (130) and the further joints (140; 150) by appropriately coordinating and adjusting the actuation paths of the blocking device (132), the transmission devices (111, 112; 121, 122) and the rotatable clamping body (141; 151).

13. The positioning arm (100) according to any one of the preceding claims, wherein at least one spring element (134) counteracting the closing force of the blocking device (132) is arranged in the region of the central joint (130).

14. The positioning arm (100) according to any one of the preceding claims, wherein at least one spring element (135) preloading at least one of the arm elements (110; 120) into a predetermined basic position is arranged in the region of the central joint (130).

15. The positioning arm (100) according to any one of claims 3 to 14, wherein the clamping body (141; 151) is connected by means of a tie rod to a connecting joint (113; 123) connecting two pincer arms (111, 112; 121, 122).

## Revendications

1. Bras de positionnement (100) pour positionner des instruments, des appareils ou des outils,
dans lequel le bras de positionnement (100) présente au moins un premier élément de bras (110) et un deuxième élément de bras (120), qui sont reliés l'un à l'autre de manière pivotante autour d'un axe de pivotement (131) au moyen d'une articulation centrale (130),
dans lequel au moins l'un des éléments de bras (110, 120) présente une articulation supplémentaire (140, 150) à une extrémité opposée à l'articulation centrale (130), dans lequel le bras de positionnement (100) présente un dispositif de blocage (132) pour bloquer et libérer l'articulation centrale (130) et l'au moins une articulation supplémentaire (140, 150),
dans lequel le dispositif de blocage (132) est disposé au niveau de l'articulation centrale (130) et provoque le blocage et la libération des articulations supplémentaires (140, 150) par le biais d'au moins un dispositif de transmission (111, 112 ; 121, 122) formé au niveau des éléments de bras (110, 120),
dans lequel le dispositif de transmission (111, 112 ; 121, 122) est constitué par une paire de bras manipulateurs extérieurs et intérieurs (111, 112 ; 121, 122), qui forment en même temps également les éléments de bras (110, 120),
dans lequel les bras manipulateurs extérieurs et intérieurs (111, 112 ; 121, 122) présentent respectivement un bras de levier (1111, 1121 ; 1211, 1221) ainsi qu'un bras de préhension (1112, 1122 ; 1212, 1222),
dans lequel les bras de levier (1111, 1121 ; 1211, 1221) sont configurés pour pouvoir être reliés par leurs extrémités arrière de manière coïncidente dans la région de l'articulation centrale (130) au moyen du dispositif de blocage (132), de sorte que le bras manipulateur extérieur (121) du deuxième élément de bras (120), le bras manipulateur intérieur (112) du premier élément de bras (110), le bras manipulateur intérieur (122) du deuxième élément de bras (120) et le bras manipulateur extérieur (111) du premier élément de bras (110) soient superposés en alternance, et dans lequel les bras de préhension (1112, 1122 ; 1212, 1222) forment une partie des articulations supplémentaires (140, 150), et
**caractérisé en ce que** lors d'un rapprochement des extrémités au niveau des bras de levier (1111, 1121, 1211 et 1221), les bras de préhension (1112, 1122 ; 1212, 1222) sont rapprochés de manière simultanée.

2. Bras de positionnement (100) selon la revendication 1, dans lequel les deux éléments de bras (110, 120) présentent respectivement une articulation supplémentaire (140 ; 150), et dans lequel le dispositif de blocage (132) présente respectivement un dispositif de transmission (111, 112 ; 121, 122) vers l'articulation supplémentaire (140 ; 150).

3. Bras de positionnement (100) selon la revendication 1 ou 2, dans lequel l'articulation supplémentaire (140 ; 150) présente un corps de serrage rotatif (141 ; 151) qui est réalisé de préférence sous forme de bille (142 ; 152) et/ou sous forme de douille rotative (143 ; 153).

4. Bras de positionnement (100) selon l'une des revendications précédentes, dans lequel le dispositif de blocage (132) présente un élément d'actionnement (1322) qui est relié à un dispositif d'actionnement (1321, 1324, 1326) et qui est configuré de manière à pouvoir être déplacé dans la direction axiale de l'articulation centrale (130).

5. Bras de positionnement (100) selon la revendication 4, dans lequel le dispositif d'actionnement est formé par une poignée rotative (1321), par une poignée de serrage (1324) reliée à un excentrique (1325) ou par un moteur (1326).

6. Bras de positionnement (100) selon l'une des revendications précédentes, dans lequel l'une des articulations supplémentaires (140) est pourvue d'un dispositif de fixation (160) approprié afin d'être monté sur une table d'opération, et dans lequel une autre des articulations supplémentaires (150) est configurée de manière à pouvoir être reliée à un adaptateur (170) approprié afin de fixer différents instruments, appareils ou outils (190 ; 191 ; 192 ; 193).

7. Bras de positionnement (100) selon l'une des revendications précédentes, dans lequel les bras manipulateurs (111, 112 ; 121, 122) sont réalisés sous la forme de pièces identiques.

8. Bras de positionnement (100) selon l'une des revendications précédentes, dans lequel les bras manipulateurs (111, 112 ; 121, 122) sont réalisés à partir d'un métal léger, d'un alliage de métal léger ou d'une matière plastique.

9. Bras de positionnement (100) selon l'une des revendications précédentes, dans lequel le dispositif de blocage (132) présente des éléments d'encliquetage (1125, 1225) disposés ou réalisés au niveau du dispositif de transmission (111, 112 ; 121, 122).

10. Bras de positionnement (100) selon l'une des revendications 6 à 9, dans lequel l'adaptateur (170) présente au moins une pièce moulée (173) configurée de manière à coopérer avec un logement (181) présentant une forme complémentaire à celle d'un support (180) pour l'outil, l'appareil ou l'instrument (190 ; 191 ; 192 ; 193).

11. Bras de positionnement (100) selon la revendication 10, dans lequel la pièce moulée (173) est configurée de manière à pouvoir être fixée dans le logement (181) au moyen d'un coulisseau (184) disposé au niveau du support (180).

12. Bras de positionnement (100) selon la revendication 3, dans lequel le dispositif de blocage (132) est adapté et configuré de manière à permettre un blocage différé de l'articulation centrale (130) et des articulations supplémentaires (140 ; 150), et ce au moyen d'un ajustement et d'un réglage adéquats des courses d'actionnement du dispositif de blocage (132), des dispositifs de transmission (111, 112 ; 121, 122) ainsi que du corps de serrage rotatif (141 ; 151).

13. Bras de positionnement (100) selon l'une des revendications précédentes, dans lequel au moins un élément de ressort (134) visant à contrebalancer la force de fermeture du dispositif de blocage (132) est disposé dans la région de l'articulation centrale (130).

14. Bras de positionnement (100) selon l'une des revendications précédentes, dans lequel au moins un élément de ressort (135) précontraignant au moins l'un des éléments de bras (110 ; 120) dans une position de base prédéterminée est disposé dans la région de l'articulation centrale (130).

15. Bras de positionnement (100) selon l'une des revendications 3 à 14, dans lequel le corps de serrage (141 ; 151) est relié au moyen d'une tige de traction à une articulation de liaison (113 ; 123) reliant deux bras manipulateurs (111, 112 ; 121, 122).
